# EUROPEAN PATENT SPECIFICATION

(11) **EP 3 836 888 B1**
(45) Date of publication and mention of the grant of the patent: **03.07.2024**
(21) Application number: 19745132.1
(22) Date of filing: 25.07.2019
(51) Int. Cl.: A61K 8/26, A61Q 15/00

(54) **METHOD OF REDUCING PERSPIRATION**
VERFAHREN ZUR REDUZIERUNG VON PERSPIRATION
PROCÉDÉ DE RÉDUCTION DE LA TRANSPIRATION

(30) Priority: 14.08.2018 WO PCT/CN2018/100367; 17.09.2018 EP 18194734
(43) Date of publication of application: 23.06.2021
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: LI, Xiaoke, Shanghai, 200335 (CN); LIU, Weining, Shanghai, 200335 (CN); SUN, Yuekui, Shanghai, 200335 (CN)
(74) Representative: Whaley, Christopher
(86) International application number: PCT/EP2019/070037
(87) International publication number: WO 2020/035279

(56) References cited:
- EP-A1- 0 459 003
- GB-A- 1 482 917
- US-A- 3 255 082
- DATABASE GNPD [Online] MINTEL; 8 November 2011 (2011-11-08), anonymous: "Antiperspirant Deodorant Roll-On", XP055555321, retrieved from www.gnpd.com Database accession no. 1658243
- Anonymous: "Armpit Ecology! - One Earth Body Care", , 16 September 2016 (2016-09-16), XP055630556, Retrieved from the Internet: URL:https://www.oneearthbodycare.com/armpi t-ecology/ [retrieved on 2019-10-09]

## Description

### Field of the invention

The present invention is in the field of antiperspirant compositions, in particular antiperspirant products and usage.

### Background of the invention

The present invention relates to the use of an antiperspirant active. These actives are added to compositions to reduce perspiration upon topical application to the body, particularly to the underarm regions of the human body viz. the axilla, and sometimes even on the upper part of the body near the chest. Usually, the antiperspirant actives are salts of metals having an astringent effect, such as the salts of aluminum and/or zirconium. Since antiperspirants are used regularly, and have been used for decades, the perceived risk associated with their use is steadily rising. Therefore, there is an ever-increasing need to develop alternative antiperspirant actives which are efficacious more and skin-friendly.

JP2002145717 A (Catalysts & Chemicals Industries) discloses an antibacterial cosmetic composition which comprises fine particles composed of an antimicrobial metal component and an inorganic oxide other than the antimicrobial metal component. It is disclosed that the fine particles comprising SiO₂, Al₂O₃ and SiO₂.Al₂O₃ composite oxides are excellent in feel during use and are suitable for anti-sunburn cosmetics and the like.

DE102004014294 A1 (Sasol Germany GMBH) relates to compositions containing a mixed oxide containing aluminium and/or magnesium and a 2-alkyl branched acid and/or derivatives thereof and their use as actives against bacteria, mycota and viruses.

EP0459003 A1 (KABUSHIKIKAISHA NIPPAN KENKYUS) relates to antimicrobial deodorant compositions containing alumina as filler.

US3255082 A (STANLEY BARTON) relates to preparation of antiperspirant stick comprising ACH and alumina is disclosed to modify the physical properties of ACH.

### Summary of the invention

The present inventors have found that colloidal particles of alumina which are amorphous in nature, are stable under low pH, i.e. from 2 to 5. In addition to being stable, the particles are capable of forming a gel when they contact with aqueous saline media, which is compositionally equivalent to perspiration or sweat. Therefore, such a gel can potentially block sweat ducts, partially or fully, in a non-permanent manner and inhibit further sweating or perspiration. Therefore, the particles can be used as actives in antiperspirant compositions, either by themselves or in combination with other conventional actives like compounds of aluminum such as ACH or zirconium. Their use in combination with other conventional actives could provide a useful technical route to reduce the reliance on conventional aluminum or zirconium based active in such compositions.

In accordance with a first aspect is disclosed the use of colloidal particles of amorphous alumina as an antiperspirant active.

All other aspects of the present invention will more readily become apparent upon considering the detailed description and examples which follow.

Except in the examples, or where otherwise explicitly indicated, all numbers in this description indicating amounts of material or conditions of reaction, physical properties of materials and/or use may optionally be understood as modified by the word "about". All amounts are by weight of the antiperspirant composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

### Detailed description of the invention

By "An antiperspirant Composition" as used herein, is meant to include a composition for topical application to the skin of mammals, especially humans. Such a composition is preferably of the leave-on type. By a leave-on composition is meant a composition that is applied to the desired skin surface and left on for one minute to 24 hours after which it may be wiped or rinsed off with water, usually during the regular course of personal washing. The composition of the present invention can be in the form of a liquid, lotion, cream, foam, scrub, gel or stick form and may be delivered through a roll-on device or using a propellant containing aerosol can. It is especially useful for delivering low pH compositions to the axilla of an individual for antiperspirant benefits. "Skin" as used herein is meant to include skin on any part of the body (e.g., neck, chest, back, arms, underarms, hands, legs, buttocks and scalp), especially the underarms.

Herein, 'antiperspirant active' should be understood to mean a substance that by itself has the effect of reducing or limiting human perspiration.

### Amorphous alumina in form of colloidal particles

The amorphous alumina which is in form of colloid particles may be used to give antiperspirancy in the absence of any other agent capable of giving this benefit.

Amorphous materials, which are different from the crystalline materials, lack the long-range order that is characteristic of a crystal. Amorphous materials have an internal structure made of interconnected structural blocks. Whether a material is liquid or solid depends primarily on the connectivity between its elementary building blocks so that solids are characterized by a high degree of connectivity whereas structural blocks in fluids have lower connectivity.

Colloidal refers to items of a small size that are floating in a medium of one of three substances: a solid, a liquid, or a gas. Colloidal particles are microscopic solid particles suspended in a fluid. Unlike a solution, whose solute and solvent constitute only one phase, a colloid has a dispersed phase (the suspended particles) and a continuous phase (the medium of suspension).

Preferably, the amorphous alumina is a cationic hydroxylated polynuclear alumina species. More preferably the amorphous alumina is an alumina sol.

A preferred amorphous alumina in form of colloidal particles is available under the name A4 Alumina Sol from Dalian Snowchemical S&T Co., Ltd. It is dispersed in aqueous medium and is mainly used in the electronics industry.

It is particularly preferred that particle size of the colloidal particles is 1 to 500 nm, more preferably 5 to 300 nm, and most preferably 10 to 200 nm. Without wishing to be bound by theory it is believed that colloidal particles with smaller size are more stable and can diffuse into sweat ducts easily as the sweat ducts have the size of around 5 to 50 microns.

When the colloidal particles of amorphous alumina is applied from a cosmetic composition, as it typically is, its level in the cosmetic composition is from 1 to 30 wt%, and more preferably from 5 to 30 wt%, and most preferably from 8 to 25 wt%, based on total weight of the composition.

Alternatively, the colloidal particles of amorphous alumina may be used with another antiperspirant active. The other antiperspirant active could be a conventional antiperspirant salt comprising Al and/or other forms of Zr, such as aluminum chlorohydrate or aluminum-zirconium chlorohydrate optionally complexed with glycine.

Without wishing to be bound by theory the inventors believe that once inside the sweat glands, due to the presence of sweat and/or sebum, the pH of the surrounding medium increases to about more than 5 to 7 as the active comes in contact with sweat. This contact would induce growth of the colloidal particles of the amorphous alumina to thereby form a gel-network which partially or fully, and in a non-permanent manner, clogs or blocks the sweat ducts to provide antiperspirant benefits.

### Other ingredients

Other components commonly included in conventional antiperspirant compositions may also be present, along with the colloidal particles of amorphous alumina. Such components include skin care agents such emollients, humectants and skin barrier promoters; skin appearance modifiers such as skin lightening agents and skin smoothing agents; anti-microbial agents, in particular organic anti-microbial agents, and preservatives.

The antiperspirant compositions are applied cosmetically and topically to the skin, broadly speaking, by one of two methods. Different consumers prefer one method or the other. In one method, sometimes called a contact method, the composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of a dispenser, such as a pump or a squeezable sidewall or by internally generated pressure arising from a fraction of a liquefied propellant volatilizing, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

A highly preferred additional component used along with the colloidal particles of amorphous alumina is a cosmetically acceptable carrier, particularly when the colloidal particles of amorphous alumina are dispersed in the carrier. The carrier can be anhydrous or aqueous. The term anhydrous implies that the composition comprises not more than 5 wt% water. The term aqueous means that the composition of the invention comprises water as the main carrier or that water forms a major part of the carrier. In such cases, other solvents and ingredients other than water may also be present. It is more preferred that the carrier is aqueous.

Preferably when the carrier is anhydrous, it comprises a silicone compound, a volatile alcohol or a wax. The silicone compound, which preferably is a volatile silicone oil is commonly cyclomethicone. Cyclomethicone is nominally designated as D4, D5 or D6, depending upon the particular cyclomethicone (e.g., cyclotetrasiloxane, cyclopentasiloxane or cyclohexasiloxane) predominant therein. Cyclomethicone is compatible with numerous carrier oils as well as with amido gelators and emulsifiers, in particular, the silicone based emulsifiers commonly employed in such compositions. Volatile silicone oils are generally considered to impart good sensory feel to such formulations, i.e., a clean, dry feel. Additionally, the surface tension and spreadability of cyclomethicone can contribute to the emulsion sticks in which they are used having a smooth or silky feel on application.

Volatile linear polydimethylsiloxanes, often referred to as volatile dimethicones, such as, for example, those sold by Dow Corning with the names DC200 Fluid 1cst and DC200 Fluid 5cst, are in some cases used as complete or partial alternatives to the cyclomethicones. Non-silicone volatile oils may also be employed for similar sensory purposes. These include linear and branched hydrocarbons containing less than about 16 carbon atoms.

It is preferred that the colloidal particles of amorphous alumina are dispersed in the carrier which is in the form of a cream, a spray, a firm solid, a soft solid or is an emulsion packaged in a roll-on applicator.

Further preferably, when said carrier is a spray it comprises a propellant and the carrier is in the form of an aerosol.

### Stick compositions

In one aspect, the antiperspirant composition of the invention is a stick composition which is usually in the form of an emulsion. Antiperspirant emulsion sticks are solidified compositions characterized as having aqueous and oil phases. Among such antiperspirant emulsion sticks are compositions having a dispersed aqueous phase in which is dissolved an antiperspirant active, in usual cases, aluminium, zirconium and/or mixed aluminium/zirconium salts, and a continuous oil phase comprising one or more gelling agents capable of structuring such phase.

Antiperspirant emulsion sticks can be formulated as clear (i.e., translucent or transparent) or opaque compositions. Translucent or transparent emulsion sticks go on clear and, depending upon their formulation, may remain clear for extended periods of time, reducing the consumer perceived negative of "white marks" associated with deposition of antiperspirant active. Different materials have been proposed as gellants for a continuous oil phase, including waxes, small molecule gelling agents and polymers. They each have their advantages and of them, one of the most popular class of gellants is waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellants for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

Stick compositions are usually available in the form of a firm solid or a soft solid. Firm solids, as the name indicates, are harder and can be directly applied by way of an applicator, for example, to the underarms. Soft solids also need an applicator which is similar to the firm solids, the difference being that the soft solids are softer and the applicator needs to be designed in order to permit extrusion of the solids through a cap member comprising plurality of orifices and the extruded composition can then be applied to the underarms.

### Roll-on

Alternatively, the composition of the invention is a liquid composition, that can be dispensed from a roll-on package. Broadly speaking such compositions could be divided into two classes, namely those in which an antiperspirant active is suspended in a hydrophobic carrier, such as a volatile silicone and those in which the antiperspirant active is dissolved in a carrier liquid. The latter is more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other depilatory actions.

The second category of formulations that is an alternative to alcoholic formulations comprise a dispersion of water-insoluble or very poorly water-soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

Further alternatively, the antiperspirant composition of the invention is delivered through an aerosol composition which comprises a propellant in addition to the applicable other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is preferred.

Propellants herein generally are one of three classes; (i) low boiling-point gasses liquified by compression, (ii) volatile ethers and (iii) compressed non-oxidising gases.

Class (i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane.

The class (ii) of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses.

The class (iii) of propellant comprises compressed non-oxidising gasses, and in particular carbon dioxide or nitrogen. Inert gases like neon are a theoretical alternative.

The composition of the present invention can comprise a wide range of other optional components. The CTFA Personal Care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, other than the ones already discussed earlier, preservatives, natural extracts, essential oils, skin sensates, skin soothing agents, and skin healing agents.

### Method and Use

The invention provides for use of colloidal particles of amorphous alumina as an antiperspirant active. The use is preferably non-therapeutic in nature, more preferably cosmetic in nature. Particularly, the present invention provides for use of colloidal particles of amorphous alumina to partially or fully block sweat ducts in a non-permanent manner.

The invention will now be demonstrated with the help of the following non-limiting examples.

### Examples

### Materials

**Table -1**

| Trade/Chemical name | Supplier | Particle size | Amount of amorphous Al₂O₃ (wt%) | pH |
|---|---|---|---|---|
| A-4 Alumina Sol | Dalian Snowchemical S&T Co., Ltd, China | 10 to 200 nm | 10 to 15 (aqueous dispersion), balance being water | 3.5 to 4.5 |

### Example 1: Gelation of composition triggered by increase in pH

The ability of the A-4 Alumina Sol to form a gel under higher pH was tested by the following procedure:
10 ml of the A-4 Alumina Sol was taken in a beaker and an aqueous solution of sodium bicarbonate was added dropwise under stirring, to increase the pH of the medium beyond 5. Meanwhile, 10 ml of the A-4 Alumina Sol was taken in a beaker and an aqueous solution of hydrochloric acid was added dropwise under stirring, to modulate the pH of the medium to be lower. The appearance of the sample was checked visually and recorded as the pH increased gradually. The sample was graded as either Semi-Transparent (ST), or Gelation (G). The data on the appearance of the sample is summarized in Table -2 below:

**Table -2**

| Reference no. | Composition | pH =2 (with HCl) | pH = 3 (with HCl) | pH = 4.25 | pH = 5.5 (with addition of NaHCO₃) |
|---|---|---|---|---|---|
| 1 | A-4 Alumina Sol | ST | ST | ST | G |

The data in Table -2 above indicates that colloidal particles of amorphous alumina as per the invention (Reference No. 1) was stable (semi-transparent) at pH 2, 3, 4.25 and its gelation was triggered by an increase in the pH of the medium (pH = 5.5). It indicates the ability of the colloidal particles of amorphous alumina as per the invention to form a gel upon contact with model sweat whilst being semi-transparent (non-gelled) at pH of 2 to 5, which is the pH at which such antiperspirant compositions are usually formulated.

## Claims

1. The use of colloidal particles of amorphous alumina as an antiperspirant active.

2. The use as claimed in claim 1 wherein particle size of said colloidal particles is 1 to 500 nm.

3. The use as claimed in claims 1 or 2 wherein said particle size is 5 to 300 nm.

4. The use as claimed in any of claims 1 to 3 wherein said colloidal particles are dispersed in a cosmetically acceptable carrier.

5. The use as claimed in claim 4 wherein level of said amorphous alumina is 1 to 30 wt% of total weight of said amorphous alumina and said carrier.

6. The use as claimed in claims 4 or 5 wherein said carrier is anhydrous, it comprises a silicone compound, a volatile alcohol or a wax.

7. The use as claimed in any of claims 4 to 6 wherein said carrier is in the form of a cream, a spray, a firm solid, a soft solid or is an emulsion packaged in a roll-on applicator.

8. The use as claimed in claim 7 wherein when said carrier is a spray, said carrier comprises a propellant and it is in the form of an aerosol.

9. The use as claimed in any of claims 4 to 8 wherein pH of said carrier is from 2 to 5.

10. The use as claimed in any of claims 1 to 9 for reduction of bodily perspiration.

11. The use as claimed in claim 1 to 10 wherein said use is non-therapeutic in nature.

12. The use as claimed in claim 1 to 11 wherein said use blocks sweat ducts, partially or fully, in a non-permanent manner.

## Patentansprüche

1. Verwendung kolloidaler Partikel aus amorphem Aluminiumoxid als schweißhemmender Wirkstoff.

2. Verwendung, wie im Anspruch 1 beansprucht, wobei die Partikelgröße der kolloidalen Partikel 1 bis 500 nm beträgt.

3. Verwendung, wie in den Ansprüchen 1 oder 2 beansprucht, wobei die Partikelgröße 5 bis 300 nm beträgt.

4. Verwendung, wie in einem der Ansprüche 1 bis 3 beansprucht, wobei die kolloidalen Partikel in einem kosmetisch akzeptablen Träger dispergiert sind.

5. Verwendung, wie im Anspruch 4 beansprucht, wobei der Anteil des amorphen Aluminiumoxids 1 bis 30 Gew.-% des Gesamtgewichts des amorphen Aluminiumoxids und des Trägers beträgt.

6. Verwendung, wie in den Ansprüchen 4 oder 5 beansprucht, wobei der Träger wasserfrei ist, eine Silikonverbindung, einen flüchtigen Alkohol oder ein Wachs umfasst.

7. Verwendung, wie in einem der Ansprüche 4 bis 6 beansprucht, wobei der Träger in Form einer Creme, eines Sprays, eines harten Feststoffs, eines weichen Feststoffs oder als in einem Roll-on-Applikator verpackte Emulsion vorliegt.

8. Verwendung, wie im Anspruch 7 beansprucht, wobei der Träger, wenn der Träger als Spray vorliegt, ein Treibmittel umfasst und in Form eines Aerosols vorliegt.

9. Verwendung, wie in einem der Ansprüche 4 bis 8 beansprucht, wobei der pH-Wert des Trägers 2 bis 5 beträgt.

10. Verwendung, wie in einem der Ansprüche 1 bis 9 beansprucht, zur Verringerung des Körperschwitzens.

11. Verwendung, wie in Anspruch 1 bis 10 beansprucht, wobei die Verwendung nicht-therapeutischer Natur ist.

12. Verwendung, wie in Anspruch 1 bis 11 beansprucht, wobei die Verwendung die Schweißdrüsen teilweise oder vollständig auf nicht-dauerhafte Weise blockiert.

## Revendications

1. Utilisation de particules colloïdales d'alumine amorphe en tant que principe actif antitranspirant.

2. Utilisation selon la revendication 1, dans laquelle la granulométrie desdites particules colloïdales est de 1 à 500 nm.

3. Utilisation selon la revendication 1 ou 2, dans laquelle ladite granulométrie est de 5 à 300 nm.

4. Utilisation selon l'une quelconque des revendications 1 à 3, dans laquelle lesdites particules colloïdales sont dispersées dans un véhicule acceptable du point de vue cosmétique.

5. Utilisation selon la revendication 4, dans laquelle le taux de ladite alumine amorphe est de 1 à 30 % en poids du poids total de ladite alumine amorphe et dudit véhicule.

6. Utilisation selon la revendication 4 ou 5, dans laquelle ledit véhicule est anhydre, comprend un composé siliconé, un alcool volatil ou une cire.

7. Utilisation selon l'une quelconque des revendications 4 à 6, dans laquelle ledit véhicule est sous la forme d'une crème, d'un spray, d'un solide dur, d'un solide mou, ou est une émulsion conditionnée dans un applicateur à bille.

8. Utilisation selon la revendication 7, dans lequel, quand ledit véhicule est un spray, ledit véhicule comprend un propulseur et est sous la forme d'un aérosol.

9. Utilisation selon l'une quelconque des revendications 4 à 8, dans laquelle le pH dudit véhicule est de 2 à 5.

10. Utilisation selon l'une quelconque des revendications 1 à 9, pour la réduction de la transpiration corporelle.

11. Utilisation selon l'une quelconque des revendications 1 à 10, laquelle utilisation est de nature non thérapeutique.

12. Utilisation selon l'une quelconque des revendications 1 à 11, laquelle utilisation bloque les canaux sudoripares, partiellement ou complètement, d'une manière non permanente.
